(19) Europäisches Patentamt / European Patent Office / Office européen des brevets

(11) **EP 3 948 894 B1**

(12) **EUROPEAN PATENT SPECIFICATION**

(45) Date of publication and mention
of the grant of the patent:
**29.07.2026 Bulletin 2026/31**

(21) Application number: **20714213.4**

(22) Date of filing: **27.03.2020**

(51) International Patent Classification (IPC):
**G16H 50/30** (2018.01)

(52) Cooperative Patent Classification (CPC):
**G16H 50/30**

(86) International application number:
**PCT/EP2020/058697**

(87) International publication number:
**WO 2020/201077 (08.10.2020 Gazette 2020/41)**

(54) **COMPUTER-IMPLEMENTED METHOD AND SYSTEM FOR DETERMINING A RELATIVE RISK FOR LACK OF GLYCEMIC CONTROL FOR A PLURALITY OF PATIENTS, AND COMPUTER PROGRAM PRODUCT**

COMPUTERIMPLEMENTIERTES VERFAHREN UND SYSTEM ZUM BESTIMMEN EINES RELATIVEN RISIKOS FÜR MANGELNDE GLYKÄMISCHE KONTROLLE BEI EINER VIELZAHL VON PATIENTEN SOWIE COMPUTERPROGRAMMPRODUKT

PROCÉDÉ ET SYSTÈME MIS EN ŒUVRE PAR ORDINATEUR PERMETTANT DE DÉTERMINER UN RISQUE RELATIF RÉSULTANT D'UNE ABSENCE DE CONTRÔLE GLYCÉMIQUE POUR UNE PLURALITÉ DE PATIENTS ET PRODUIT-PROGRAMME D'ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2019 EP 19166408**

(43) Date of publication of application:
**09.02.2022 Bulletin 2022/06**

(73) Proprietor: **mySugr GmbH
1010 Wien (AT)**

(72) Inventors:
• **BIVEN, Richard P.**
**1010 Wien (AT)**
• **SCHUSTER, Lukas**
**1010 Wien (AT)**
• **THEISSEN, Michèle**
**1010 Wien (AT)**
• **WREDE, Jan**
**1010 Wien (AT)**

(74) Representative: **Wandrey, Anne Julia
Roche Diagnostics GmbH
LPDD...6918
Sandhofer Straße 116
68305 Mannheim (DE)**

(56) References cited:
EP-A1- 2 339 953          EP-B1- 2 339 953
WO-A1-2017/030961          US-A1- 2011 264 378
US-A1- 2012 172 694          US-A1- 2013 102 867
US-A1- 2014 188 400

• B. BUIJSSE ET AL: "Risk Assessment Tools for Identifying Individuals at Risk of Developing Type 2 Diabetes", EPIDEMIOLOGIC REVIEWS, vol. 33, no. 1, 27 May 2011 (2011-05-27), pages 46 - 62, XP055276171, ISSN: 0193-936X, DOI: 10.1093/epirev/mxq019

**Description**

The present disclosure refers to a computer-implemented method and a system for determining a relative risk for lack of glycemic control for a plurality of patients. Also, a computer program product is referred to.

Background

Glucose measurement data collected for one or more patients may be analyzed for providing caregivers information as whether some patient needs assistance or support. There are different types of measurements conducted for gathering glucose measurement data, such as blood glucose measurement data. Also, continuous glucose monitoring (CGM) may be applied. For such type of measurement the frequency of measurement events ($1 / T$) is very high, with typical measurement time interval T approximately ranging from 5 to 15 minutes, which allows analyses with high resolution. In contrast, for patients performing so-called spot monitoring (blood) glucose measurements (SPM), the number of samples per day is comparably low, e.g., around 3 to 8 samples, and mostly unevenly distributed in time. Spot monitoring blood glucose (SPM) measurement may also be referred to as self-monitored blood glucose (SMBG) measurement.

Document US 2009 / 0171589 A1 describes computing an average daily risk range (ADRR). Parameters low blood glucose index (LBGI) and high blood glucose index (HBGI) are referred to in this document with indication of further references.

Document US 2015 / 0095042 A1 discloses classifying a patient as having a first, second or third risk of being hypoglycemic in the future based on a value (e.g. LBGI value) which is calculated based on blood glucose levels and displaying the classification on a display. Also, it may be determined whether a pattern is present in a patient's blood glucose data. The order of display of recognized patterns may be prioritized.

Document EP 5 062 615 B1 refers to a system for monitoring, diagnosing and treating medical conditions of a plurality of remotely located patients comprising a central data processing system, means for obtaining patient data from remotely located patient monitoring systems, means for analyzing the obtained patient data from each respective patient monitoring system to identify medical conditions of each respective patient; means for displaying identified patient medical conditions for each respective patient in selectable prioritized order according to medical severity. Communication between portable patient monitors (PPMs) with a central data processing system referred to as a Physicians Access Center server ("PAC server") is described.

Document WO 2016 / 182972 A1 discloses categorization of a plurality of patients into diabetes risk categories for caregivers.

Document US 2006 / 0253303 A1 relates to providing a multiple patient monitoring system. An overview chart is provided, wherein each data point represents one corresponding patient and indicates the control value, e.g. a mean value, and the elapsed time period since the collection date of' the set of measurements most recently collected for the patient. Furthermore, each icon may indicate compliance of the corresponding patient with the prescribed measurement regimen. The clinician can thus view and manage the medical priorities of an entire group of patients simultaneously. It also allows the clinician to communicate proactively with unmotivated patients who have lost contact with the clinician before these patients develop urgent medical needs.

Document WO 2017 / 030961 A1 discloses a patient prioritization module which sorts and prioritizes the healthcare provider's patients based on the alert status of each of the patient. The alert sort algorithm pre-assigns a weight factor for each glucose condition and for each possible state of a given glucose condition.

Document WO 2007 / 081853 A2 pertains to a system, a computer program product, a method and an algorithm for the evaluation of blood glucose. The method employs routine self-monitoring blood glucose data collected over a period of 2-6 weeks, based on a theory of risk analysis of blood glucose data. An Average Daily Risk Range (ADRR) is computed as a measure of overall glucose variability. Further, the glucose variability in the hypoglycemic range via a Low BG Index (LBGI) and the glucose variability in the high BG range via High BG Index (HBGI) followed by a combination of the two indices into a single variability display may be estimated separately.

The document Kovatchev et al., Risk analysis of blood glucose data: a quantitative approach to optimizing the control of insulin dependent diabetes, J. Theor. Med. 3, 1-10 (2000), pertains to quantitative tools for online assessment of the quality of an optimization based on self-monitoring of blood glucose.

The document Kovatchev, Metrics for glycemic control from HbA1c to continuous glucose monitoring, Nature Rev. Endocrinology 13, 425-436 (2017) refers to the assessment, quantification and optimal control of glucose fluctuations in diabetes mellitus, focusing on markers of average glycaemia and the utility and shortcomings of HbA1c as a gold-standard metric of glycemic control.

The document Kovatchev et al., Evaluation of a New Measure of Blood Glucose Variability in Diabetes, Diabetes Care 29:11, 2433-2438 (2006) pertains to average daily risk range (ADRR) as a variability measure computed from routine self-monitored blood glucose (SMBG) data.

**EP 3 948 894 B1**

The document US 2013 0102867 A1 relates to techniques to determine a metric quantifying a glycemic health of a patient. The document US 2014 0188400 A1 relates to a system and method for collecting, analyzing, and displaying analyses of medical analyte data for managing diabetes mellitus. The document EP 2339953 A1 relates to systems and methods for evaluating glycemic control of a patient. The document Buijsse et al., Risk Assessment Tools for Identifying Individuals at Risk of Developing Type 2 Diabetes, Epidemiologic Review 33:1, 46-62 relates to the assessment of risk scores to predict future diabetes.

The documents do not disclose an assignment of evaluation parameters to certain glucose measurement frequencies for calculation of total risk scores derived from a plurality of evaluation parameters for the determination of a lack of glycemic control of patients and/or do not disclose electronic messages being sent to patients comprising a prompt to increase the measurement frequency depending on the total risk scores.

Summary

It is an object of the present disclosure to provide a computer-implemented method and a system for determining a relative risk for lack of glycemic control for a plurality of patients, wherein the method and the system can be applied for reliable determination of the relative risk for lack of glycemic control for glucose measurement data collected by conducting different measurement conditions.

The present invention is as defined in the claims.

According to an aspect, a computer-implemented method for determining a relative risk for lack of glycemic control for at least two patients is provided, the method, in a data processing device having one or more data processors and a data storage device connected to the one or more data processors, comprising: providing a first set of glucose measurement data assigned to at least one a first patient, the first set of glucose measurement data collected by conducting a first frequency of measurement events; providing a second set of glucose measurement data assigned to a second patient, the second set of glucose measurement data collected by conducting a second frequency of measurement events which is different from the first frequency of measurement events; providing a plurality of evaluation parameters in the data storage device, each of the evaluation parameters assigned to at least one of the first frequency of measurement events and the second frequency of measurement events; determining, depending on the first frequency of measurement events, a plurality of first evaluation parameters from the plurality of evaluation parameters in the data storage device, wherein the plurality of first evaluation parameters is selected from the following group of evaluation parameters: low blood glucose index; high blood glucose index, mean blood glucose, and median blood glucose; determining, depending on the second frequency of measurement events, a plurality of second evaluation parameters from the plurality of evaluation parameters in the data storage device, wherein at least one of the determined plurality of second evaluation parameters is different from the plurality of first evaluation parameters, and wherein the plurality of second evaluation parameters is selected from the following group of evaluation parameters: low blood glucose index; high blood glucose index, stability index defined as the mean value of the second set of glucose measurement data divided by the standard deviation, and average daily risk range (ADRR); determining, from the first set of glucose measurement data, a plurality of first evaluation parameter values assigned to the first patient for the plurality of first evaluation parameters; determining, from the second set of glucose measurement data, a plurality of second evaluation parameter values assigned to the second patient for the plurality of second evaluation parameters; determining a plurality of first risk scores assigned to the first patient and a plurality of second risk scores assigned to the second patient from the plurality of first evaluation parameter values and the plurality of second evaluation parameter values, respectively; and determining a relative risk for lack of glycemic control by comparing a first total risk score and a second total risk score, wherein the first total risk score and the second total risk score are determined by summing up first and second risk scores for the plurality of first evaluation parameter values and the plurality of second evaluation parameter values, respectively, the relative risk indicating the risk for lack of glycemic control being higher for one of the first patient and the at least one second patient; the method further comprising at least one of the following: a) predefining a first maximum achievable total risk score and a first threshold value, and a ratio of the first total risk score and the first maximum achievable total risk score being above the first predefined threshold triggering an electronic message being sent to the first patient comprising a prompt to increase the measurement frequency, wherein the first predefined threshold is above 0.5, preferably above 0.7, more preferably above 0.8 or 0.9; and b) predefining a second maximum achievable total risk score and a second threshold value, and a ratio of the second total risk score and the second maximum achievable total risk score being above the second predefined threshold, triggering an electronic message being sent to the second patient comprising a prompt to increase the measurement frequency, wherein the second predefined threshold is above 0.5, preferably above 0.7, more preferably above 0.8 or 0.9.

According to another aspect, a system for determining a relative risk for lack of glycemic control for at least two patients is provided, the system comprising a data processing device having one or more data processors and a data storage device connected to the one or more data processors, wherein the data processing device is configured to: provide a first set of glucose measurement data assigned to a first patient, the first set of glucose measurement data collected conducting a first frequency of measurement events; provide a second set of glucose measurement data assigned to at

3

least one a second patient, the second set of glucose measurement data collected conducting a second frequency of measurement events which is different from the first frequency of measurement events; provide a plurality of evaluation parameters in the data storage device, each of the evaluation parameters assigned to at least one of the first frequency of measurement events and the second frequency of measurement events; determine, depending on the first frequency of measurement events, a plurality of first evaluation parameters from the plurality of evaluation parameters in the data storage device, wherein the plurality of first evaluation parameters is selected from the following group of evaluation parameters: low blood glucose index; high blood glucose index, mean blood glucose, and median blood glucose; determine, depending on the second frequency of measurement events, a plurality of second evaluation parameters from the plurality of evaluation parameters in the data storage device, wherein at least one of the determined plurality of second evaluation parameters is different from the plurality of first evaluation parameters, and wherein the plurality of second evaluation parameters is selected from the following group of evaluation parameters: low blood glucose index; high blood glucose index, stability index defined as the mean value of the second set of glucose measurement data divided by the standard deviation, and average daily risk range (ADRR); determine, from the first set of glucose measurement data, a plurality of first evaluation parameter values assigned to the first patient for the plurality of first evaluation parameters; determine, from the second set of glucose measurement data, a plurality of second evaluation parameter values assigned to the second patient for the plurality of second evaluation parameters; determine a plurality of first risk scores assigned to the first patient and a plurality of second risk scores assigned the second patient from the plurality of first evaluation parameter values and the plurality of second evaluation parameter values, respectively; and determine a relative risk for lack of glycemic control by comparing a first total risk score and a second total risk score, wherein the first total risk score and the second total risk score are determined by summing up first and second risk scores for the plurality of first evaluation parameter values and the plurality of second evaluation parameter values, respectively, the relative risk indicating the risk for lack of glycemic control being higher for one of the first patient and the second patient; the data processing device further being configured to at least one of the following: a) trigger an electronic message being sent to the first patient comprising a prompt to increase the measurement frequency if a ratio of the first total risk score and a predefined first maximum achievable total risk score is above a first predefined threshold, wherein the first predefined threshold is above 0.5, preferably above 0.7, more preferably above 0.8 or 0.9; and b) trigger an electronic message being sent to the second patient comprising a prompt to increase the measurement frequency if a ratio of the second total risk score and a predefined second maximum achievable total risk score is above a second predefined threshold, wherein the second predefined threshold is above 0.5, preferably above 0.7, more preferably above 0.8 or 0.9.

Further, a computer program product is provided, comprising program code configured to, when loaded into a computer having one or more processors, perform the implemented method for determining a relative risk for lack of glycemic control for at least two patients as described herein.

The relative risk indicating the risk for lack of glycemic control may give indication that a patient or a group of patients is in higher or more urgent need for assistance or coaching than some other patient or group of patients due to different risk score. Such information can be provided to some health care provider or professional, for example, by outputting audio and / or video data indicating the relative risk for lack of glycemic control.

The first set of glucose measurement data and the second set of glucose measurement data may be received from at least one of spot monitoring blood glucose (SPM) measurement which also be referred to as self-monitored blood glucose (SMBG) measurement and continuous glucose monitoring (CGM). The data processing device may, e.g., receive the first set of glucose measurement data and the second set of glucose measurement data wirelessly from handheld portable glucose meters (for making spot monitoring blood glucose measurements) and / or from continuous glucose monitoring devices (for continuous glucose monitoring).

For determining the first evaluation parameter value all measurement data or a subset of measurement data from the first set of glucose measurement data provided may be analyzed. The same applies to the determining of the second evaluation parameter value, analysis of all measurement data or a subset of measurement data from the second set of glucose measurement data provided may be conducted. For example, no data older than 30 days or older than 14 days may be analyzed for determining the relative risk for lack of glycemic control.

In one embodiment, the first set of measurement data may comprise or consist of data not older than 30 days or not older than 14 days, and the second set of measurement data may comprise or consist of data not older than 30 days or not older than 14 days for determining the relative risk for lack of glycemic control.

Different frequencies of measurement events are assigned different sets of evaluation parameters in the data storage device. Such assignment may be provided by assignment data stored in the data storage device. The assignment data may be stored in the data storage device in response to a user input indicating the assignment. Alternatively, the assignment data may be received from some external source such as a remote server device or a remote input device. After receiving the assignment data in a processor of the data processing device, the evaluation parameter(s) assigned to the first and the second frequency of measurement events may be determined from the assignment data..

The method may comprise at least one of the following: providing a first set of glucose measurement data collected in a first plurality of spot monitoring glucose measurement events applying the first frequency of measurement events; and providing a second set of glucose measurement data collected in a second plurality of spot monitoring glucose measurement events applying the second frequency of measurement events.

Depending on the frequency of measurement events conducted in the glucose measurement different evaluation parameters are applied for determining the relative risk for lack of glycemic control. Still, the risk score determined for the different sets of glucose measurement data each assigned to one or more patients can be compared for determining the relative risk for lack of glycemic control. Based on such information about the relative risk some care keeper can decide whether some patient is more in need for consultation or support then some other patient.

For at least one of the first and second set of glucose measurement data the following may be provided: determining one or more consecutive glucose measurement values being collected subsequently to collecting a preceding glucose measurement value in a preceding measurement event, the one or more measurement values collected in one or more consecutive measurement events within a predetermined time window subsequently to the preceding measurement event; and excluding the one or more consecutive glucose measurement values from the determining of the first / second evaluation parameter value.. In an embodiment, the predetermined time window may be about 30 min or 60 min.

In the data storage device time window data may be stored, for example, in response to a user input indicating a time window. In the process of determining the one or more consecutive glucose measurement values, the time window data may be retrieved by one of the processors from the data storage device. Following, the time window defined by the time window data is determined by the processor and applied. In addition or as an alternative, number data may be stored in the data storage device, the number data being indicative of a predetermined number of consecutive glucose measurement values to be excluded. The number data may be received in the one or more processors from the data storage device and applied in the process of excluding the one or more consecutive glucose measurement values.

At least one common evaluation parameter may be provided in both the plurality of first and the plurality of second evaluation parameters.

The method may provide the following: the first set of glucose measurement data is assigned to a first patient having a first type of diabetes; and the second set of glucose measurement data is assigned to a second patient having a second type of diabetes. Further, the method may comprise selecting at least one of the at least one first evaluation parameter and the at least one second evaluation parameter depending on the first type of diabetes and the second type of diabetes, respectively. In the data storage device additional assignment data may be stored, for example, in response to a user input indicating assignment of the first set of glucose measurement data to the first patient having the first type of diabetes, and assignment of the second set of glucose measurement data to the second patient having the second type of diabetes. In addition or as an alternative, the additional assignment data may be indicative of the assignment between the different types of diabetes on one side and different sets of evaluation parameters on the other side. In the process of determining the risk score(s), the additional assignment data may be retrieved from the data storage device. Following, the assignment of evaluation parameter(s) defined by the additional assignment data is determined from the additional assignment data by a processor of the data processing device and applied.

The second patient may be different from the first patient. Alternatively the first and second set of glucose measurement data may be collected for one and the same patient, but will refer to different measurement time windows for such patient. In such alternative example the first and second risk scores refer to an analysis of glucose measurement data for one and same person (patient), but the first and second measurement data collected at different measurement time windows for such patient who is referred to as first and second patient for differentiating the first and second measurement data collected at different times.

The risk scores for the plurality of first evaluation parameter values and the plurality of second evaluation parameter values, respectively, may be determined according to Table 1.

Table 1

| Risk scores | 0 (NO RISK) | 1 (LOW RISK) | 2 (MEDIUM RISK) | 3 (HIGH RISK) |
|---|---|---|---|---|
| LBGI | $\leq 1$ | $>1$ & $\leq 2.5$ | $>2.5$ & $\leq 5$ | $>5$ |
| HBGI | $\leq 1$ | $>1$ & $\leq 4.5$ | $>4.5$ & $\leq 9$ | $>9$ |
| SI | $\geq 3$ | $<3$ & $\geq 2$ | $<2$ & $\geq 1$ | $<1$ |
| ADRR | $\leq 19$ | $>19$ & $\leq 30$ | $>30$ & $\leq 40$ | $>40$ |
| Median | $\geq 70$ & $\leq 140$ | $>140$ & $\leq 154$ | $>154$ & $\leq 169$ or $<70$ | $>169$ |

In one embodiment the first patient has type 1 or type 2 diabetes and the second patient has type 1 or type 2 diabetes. In one embodiment, one of the first and second patient has type 1 diabetes and the other one has type 2 diabetes.

At least one of the first total risk score and the second total risk score, being above a predefined threshold value, may be indicative of at least one of a need for therapy review, and therapy adjustment for the first patient and the second patient, respectively.

The predefined threshold value may be stored in and retrieved from the data storage device for determining whether or not there is a need of therapy review and / or therapy adjustment and / or for triggering an automatic electronic message being sent to the patient(s) whose score(s) exceed(s) the threshold value.

The predefined first maximum achievable total risk score and the predefined first threshold value may be stored in and retrieved from the data storage device for determining whether or not there is a need of therapy review and / or therapy adjustment for the first patient and / or for triggering an automatic electronic message being sent to the first patient if the first threshold value is exceeded. The predefined second maximum achievable total risk score and the predefined second threshold value may be stored in and retrieved from the data storage device for determining whether or not there is a need of therapy review and / or therapy adjustment for the second patient and / or for triggering an automatic electronic message being sent to the second patient if the second threshold value is exceeded.

The message may also comprise one or more of i) teaching material on how to improve glycemic control and/or ii) a meeting request with a health care professional.

Output data indicating information about the relative risk may be output through a display device connected to the data processing device.

The embodiments disclosed for the method above may apply to the system *mutatis mutandis.*

Description of further embodiments

In the following, further embodiments, by way of example, are described with reference to figures. In the figures show:

Fig. 1   schematic representation of a system for determining a relative risk for lack of glycemic control for a plurality of patients;

Fig. 2   a schematic representation for a method for determining a relative risk for lack of glycemic control for a plurality of patients;

Fig. 3   a table summarizing the scoring system and comprising different risk metrics;

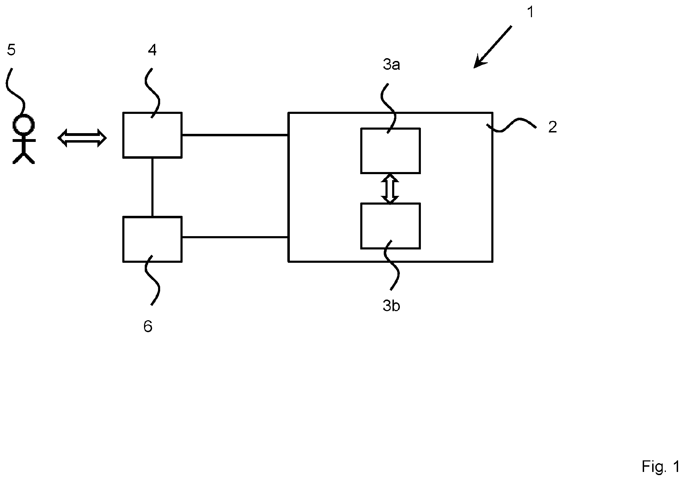

Fig. 1 shows a schematic representation of a system 1 for determining a relative risk for lack of glycemic control for a plurality of patients, the system 1 comprising a data processing device 2 having one or more data processors 3a. The data processing device 2 which may be implemented on a mobile or non-mobile device further comprises a data storage device 3b functionally connected to the one or more data processors 3a for exchanging electronic data. Form a measurement or analysis device 4, the data processing device 2 is receiving measurement data indicative of a glucose level for one or more patients 5. Measurement data indicative of a glucose level for at least two patients will be received. The measurement for different patients may be received from different measurement or analysis devices used by the different patients. For example, each patient may be using its personal measurement or analysis device for collecting the measurement data indicative of the patient's (blood) glucose level. Alternatively, the measurement data which may also be referred to as readings may be received from one or more other data processing devices 6 which received the measurement data before, for example, form a plurality of measurement or analysis devices.

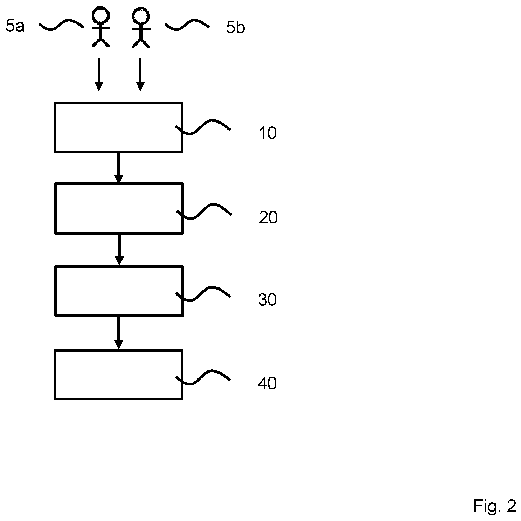

Fig. 2 shows a schematic representation for a computer-implemented method for determining a relative risk for lack of glycemic control for at least two patients 5a, 5b. The determining of the relative risk for lack of glycemic control comprises assigning risk metrics to the patients 5a, 5b. In a (optional) first step 10, measurement data collected in SMBG (self-monitoring of blood glucose) which may also be referred to as spot-monitoring (SPM) measurement are cleaned. The measurement data representing SMBG readings or measurement (events) may have to be cleaned, since some of the patients 5 may have recorded several SMBG readings within a short time period. This is sometimes referred to as "binge monitoring", which may be due to intensified control in time periods of glycemic excursion or activities that pose an increased risk such as sports. SMBG readings for one and the same patient that take place within a predetermined time period of, for example, 30 minutes or less are removed from the input data by the cleaning. Alternatively, a predetermined time period of 60 minutes may apply. Thus, the data are not skewed around such time periods.

Following, in step 20 the at least two patients 5a, 5b are grouped. For example, each patient may be assigned to one of the following groups: $G_1$ - patients that record less than two SMBG readings per day on average, and $G_2$ - users that record two or more SMBG readings per day on average. In an embodiment, the groups $G_1$ and $G_2$ may represent SMBG readings of less than 2 (or less than 5) and $\geq 2$ (or $\geq 5$), respectively, per day in 14 days out of 30 consecutive days in which most recordings or measurements were conducted. The SMBG readings of groups $G_1$ and $G_2$ represent a first frequency of measurement events and a second frequency of measurement events, respectively. In this example, the second frequency of measurement events is higher than the first frequency of measurement events.

As an example, if a patient checks their SMBG a minimum of two times per day for at least 14 days within the last 30 days, they will be assigned to the group $G_2$. Note that such measure allows for days where the patient records less frequent or not at all as long as they have at least fourteen days meeting the above requirement. Also, if another patient records at least 4 SMBG data every other day for 30 days, they will also be assigned to the group $G_2$.

Alternatively, the groups $G_1$ and $G_2$ can also represent less than 2 (or less than 5) and $\geq 2$ (or $\geq 5$) SMBG readings, respectively, per day in 7 days out of 14 consecutive days in which most recordings were performed. The two-week time span may be beneficial because it looks at only most recent measurement data. Measurement events that take place 30 days prior, might not be related to the patient's current therapy regimen or easy to remember. However, on the other hand, 7 out of 14 days is less data for analysis.

Referring to Fig. 2, in step 30 for the patients 5 in groups $G_1$ and $G_2$ risk scores are determined. Following, in step 40 a relative risk for lack of glycemic control is determined by comparing the (total) risk scores for the patients 5a, 5b assigned to groups G1 and G2.

Since the groups $G_1$ and $G_2$ are representing patients having applied different frequencies for SMBG readings or measurements, different metric(s) or a different plurality of metrics (which may have at least one metric in common) is applied for the patients in the two different groups for determining individual risk scores. The application of different metrics allows for comparing the risk score determined for the patients in groups $G_1$ and $G_2$, thereby, determining a relative risk for lack of glycemic control. Such relative risk may be higher for a patient of one or the other group depending on the actual SBMG readings or measurements. Also, more than two patients may be compared. In case of a number of i ($i > 2$) patients, the patients may be assigned to groups $G_j$ ($j \leq i$).

A first metric which may referred to as ADRR (Average Daily Risk Range) is proposed, ADRR constituting an adequate risk measure capable of comparing risks for lack of glycemic control for patients 5 falling in the different groups $G_1$ and $G_2$.

In order to calculate the risk for high and low SMBG values, HBGI (High Blood Glucose Index) and LBGI (Low Blood Glucose Index) transformation equations (2) and (3), as well as published risked values are employed (cf. Kovatchev et al. (2000)).

$$LBGI = \frac{1}{n} \sum_{i=1}^{n} rl(x_1)$$

(2)

$$HBGI = \frac{1}{n} \sum_{i=1}^{n} rh(x_1)$$

(3)

where

$$rl(BG) = \begin{cases} r(BG) & \text{if } f(BG) < 0 \\ 0 & \text{otherwise} \end{cases}$$

$$rh(BG) = \begin{cases} r(BG) & \text{if } f(BG) > 0 \\ 0 & \text{otherwise} \end{cases}$$

(4)

with BG denoting the recorded blood glucose value in a SMBG recording,

$$r(BG) = 10 \cdot f(BG)^2$$

(5)

and

$$f(\text{BG}) = 1.509 \cdot \left(\ln(\text{BG})^{1.084} - 5.381\right)$$

(6)

if the values for BG are measured in units of milligrams per deciliter, or

$$f(\text{BG}) = 1.794 \cdot \left(\ln(\text{BG})^{1.026} - 1.861\right)$$

(6')

if the values for BG are measured in units of millimoles per liter. The function r can be interpreted as a measure of the risk associated with a certain BG level. The function rl depending on BG represents a low risk and the function rh represents a high risk. The function $f$ is a continuous function defined on a BG range of 1.1 to 33.3 millimoles per liter. Its form can be obtained as described in Kovatchev et al. (2000).

LBGI and HBGI are known to provide an assessment of patients' glycemic control covering both the risk for hypoglycemia and the risk of hyperglycemia, respectively. LBGI and HBGI are not time-dependent variables. They are a transformation and normalization of SMBG values to provide an equal risk scale for hypoglycemia and hyperglycemia. They are further known to gradually increase with the extent and frequency of hypoglycemic and hyperglycemic events. Therefore, LBGI and HBGI were applied for all patients 5 in each group $G_1$ and $G_2$, allowing for comparable metrics for both low and high frequency SMBG patients 5.

Based on the LBGI and HBGI values, the metric ADRR is calculated. ADRR provides a risk assessment of the total daily blood glucose variation, i.e., the sum of peak risks of hypoglycemia and hyperglycemia events per day. The ADRR is calculated as follows:

$$\text{ADRR} = \frac{1}{M} \sum_{i=1}^{M} \left(\text{LR}^i + \text{HR}^i\right)$$

(7)

where

$$\text{LR}^i = \max\left(\text{rl}\left(x_1^i\right), \text{rl}\left(x_2^i\right), \ldots, \text{rl}\left(x_{n_i}^i\right)\right),$$

$$\text{HR}^i = \max\left(\text{rh}\left(x_1^i\right), \text{rh}\left(x_2^i\right), \ldots, \text{rh}\left(x_{n_i}^i\right)\right).$$

(8)

The number of readings for day i is denoted with $n_i$ and the blood glucose data points for day $i$ with $x_1^i, x_1^i, \ldots, x_{n_i}^i$ (cf. WO 2007 / 081853 and Kovatchev et al. (2006)). M is the total number of days for which the ADRR is calculated.

For calculating the ADRR, a number of two readings per day can be sufficient. Alternatively, a minimum of three (or five) SMBG readings is used to calculate the ADRR.

The stability index (SI) is the mean value of the patient's glucose measurement data divided by the standard deviation (SI = $\mu/\sigma$). A minimum of two SBGM readings or measurements a day is required to calculate a reasonable standard deviation. Alternatively, a minimum of three (or five) SMBG readings is used to calculate the SI.

Since both ADRR and SI require a minimum reading count for usage, two further metrics representing comparable risk for low frequency patients (group $G_1$) have been devised. These metrics will be discussed next. As HBGI and LBGI do not require a minimum number of SMBG readings, HBGI and LBGI are used for all patients 5 ($G_1$ and $G_2$) in order to find the risk of high and low glycemic excursion.

As an equivalent metric for ADRR, the median of the SMBG values of the patients 5 of the group $G_1$ can be employed. In one embodiment, the mean of the SMBG values of the patients 5 of the group $G_1$ can be employed. However, the median is less sensitive to outliers than the mean.

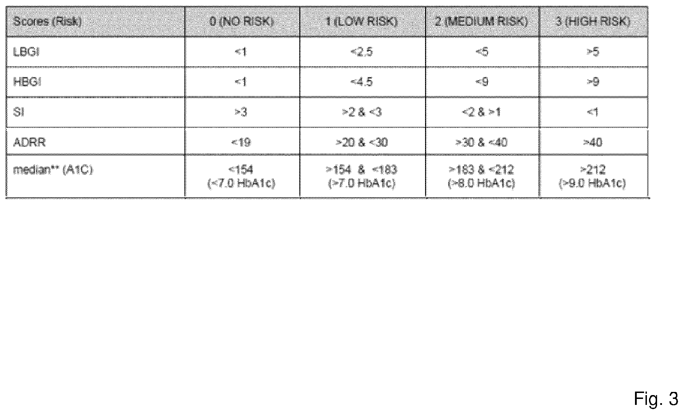

Fig. 3 shows a table summarizing the scoring system and comprising different risk metrics. For the different metrics such as LBGI, HBGI, and ADRR score criteria are depicted. Scores from 0 to 3 are applied, such scores referring to no risk (0), low risk (1), medium risk (2), and high risk (3).

In Fig. 3, A1C refers to (HbA1c). The median values for risk relate to hemoglobin A1C (HbA1C) below 7.0 %, 8.0 %, 9.0 %, and greater than 9.0 %.

An alternative scoring system is described in Table 1 above.

**Claims**

1. A computer-implemented method for determining a relative risk for lack of glycemic control for at least two patients, the method, in a data processing device (2) having one or more data processors (3a) and a data storage device (3b) connected to the one or more data processors (3a), comprising

   - providing a first set of glucose measurement data assigned to a first patient, the first set of glucose measurement data collected by conducting a first frequency of measurement events;
   - providing a second set of glucose measurement data assigned to a second patient, the second set of glucose measurement data collected by conducting a second frequency of measurement events which is different from the first frequency of measurement events;
   - providing a plurality of evaluation parameters in the data storage device (3b), each of the evaluation parameters assigned to at least one of the first frequency of measurement events and the second frequency of measurement events;
   - determining, depending on the first frequency of measurement events, a plurality of first evaluation parameters from the plurality of evaluation parameters in the data storage device (3b), wherein the plurality of first evaluation parameters is selected from the following group of evaluation parameters: low blood glucose index; high blood glucose index, mean blood glucose, and median blood glucose;
   - determining, depending on the second frequency of measurement events, a plurality of second evaluation parameters from the plurality of evaluation parameters in the data storage device (3b), wherein at least one of the determined plurality of second evaluation parameters is different from the plurality of first evaluation parameters, and wherein the plurality of second evaluation parameters is selected from the following group of evaluation parameters: low blood glucose index; high blood glucose index, stability index defined as the mean value of the second set of glucose measurement data divided by the standard deviation, and average daily risk range (ADRR);
   - determining, from the first set of glucose measurement data, a plurality of first evaluation parameter values assigned to the first patient for the plurality of first evaluation parameters;
   - determining, from the second set of glucose measurement data, a plurality of second evaluation parameter values assigned to the second patient for the plurality of second evaluation parameters;
   - determining a plurality of first risk scores assigned to the first patient and a plurality of second risk score assigned to the second patient from the plurality of first evaluation parameter values and the plurality of second evaluation parameter values, respectively; and
   - determining a relative risk for lack of glycemic control by comparing a first total risk score and a second total risk score, wherein the first total risk score and the second total risk score are determined by summing up first and second risk scores for the plurality of first evaluation parameter values and the plurality of second evaluation parameter values, respectively, the relative risk indicating the risk for lack of glycemic control being higher for one of the first patient and the second patient
   - the method further comprising at least one of the following:

      a) predefining a first maximum achievable total risk score and a first threshold value, and a ratio of the first total risk score and the first maximum achievable total risk score being above the first predefined threshold triggering an electronic message being sent to the first patient comprising a prompt to increase the measurement frequency, wherein the first predefined threshold is above 0.5, preferably above 0.7, more preferably above 0.8 or 0.9; and
      b) predefining a second maximum achievable total risk score and a second threshold value, and a ratio of the second total risk score and the second maximum achievable total risk score being above the second predefined threshold, triggering an electronic message being sent to the second patient comprising a prompt to increase the measurement frequency, wherein the second predefined threshold is above 0.5, preferably above 0.7, more preferably above 0.8 or 0.9.

2. The method according to claim 1, comprising at least one of the following:

   - providing a first set of glucose measurement data collected in a first plurality of spot monitoring glucose measurement events applying the first frequency of measurement events; and

- providing a second set of glucose measurement data collected in a second plurality of spot monitoring glucose measurement events applying the second frequency of measurement events.

3. The method according to claim 1 or 2, comprising, for at least one of the first and second glucose measurement data:

- determining one or more consecutive glucose measurement values being collected subsequently to collecting a preceding glucose measurement value in a preceding measurement event, the one or more measurement values collected in one or more consecutive measurement events within a predetermined time window subsequently to the preceding measurement event; and
- excluding the one or more consecutive glucose measurement values from the determining of the first / second evaluation parameter value.

4. The method according to claim 1, wherein at least one common evaluation parameter is provided in both the plurality first and the plurality of second evaluation parameters.

5. The method according to at least one of the preceding claims, wherein

- the first set of glucose measurement data is assigned to a first patient having a first type of diabetes; and
- the second set of glucose measurement data is assigned to a second patient having a second type of diabetes;

further comprising selecting at least one of the at least one first evaluation parameter and the at least one second evaluation parameter depending on the first type of diabetes and the second type of diabetes, respectively.

6. The method according to at least one of the preceding claims, wherein the second patient is different from the first patient.

7. The method according to at least one of the preceding claims, wherein at least one of the first total risk score and the second total risk score, being above a predefined threshold value, is indicative of at least one of a need for therapy review, and therapy adjustment for the first patient and the second patient, respectively.

8. System for determining a relative risk for lack of glycemic control for at least two patients, the system comprising a data processing device (2) having one or more data processors (3a) and a data storage device (3b) connected to the one or more data processors (3a), wherein the data processing device (2) is configured to

- provide a first set of glucose measurement data assigned to a first patient, the first set of glucose measurement data collected by conducting a first frequency of measurement events;
- provide a second set of glucose measurement data assigned to a second patient, the second set of glucose measurement data collected by conducting a second frequency of measurement events which is different from the first frequency of measurement events;
- provide a plurality of evaluation parameters in the data storage device (3b), each of the evaluation parameters assigned to at least one of the first frequency of measurement events and the second frequency of measurement events;
- determine, depending on the first frequency of measurement events, a plurality of first evaluation parameters from the plurality of evaluation parameters in the data storage device (3b), wherein the plurality of first evaluation parameters is selected from the following group of evaluation parameters: low blood glucose index; high blood glucose index, mean blood glucose, and median blood glucose;
- determine, depending on the second frequency of measurement events, a plurality of second evaluation parameters from the plurality of evaluation parameters in the data storage device (3b), wherein at least one of the determined plurality of second evaluation parameters is different from the plurality of first evaluation parameters, and wherein the plurality of second evaluation parameters is selected from the following group of evaluation parameters: low blood glucose index; high blood glucose index, stability index defined as the mean value of the second set of glucose measurement data divided by the standard deviation, and average daily risk range (ADRR);
- determine, from the first set of glucose measurement data, a plurality of first evaluation parameter values assigned to the first patient for the plurality of first evaluation parameters;
- determine, from the second set of glucose measurement data, a plurality of second evaluation parameter values assigned to the second patient for the plurality of second evaluation parameters;
- determine a plurality of first risk scores assigned to the first patient and a plurality of second risk scores assigned to the second patient from the plurality of first evaluation parameter values and the plurality of second evaluation parameter values, respectively; and

- determine a relative risk for lack of glycemic control by comparing a first total risk score and a second total risk score, wherein the first total risk score and the second total risk score are determined by summing up first and second risk scores for the plurality of first evaluation parameter values and the plurality of second evaluation parameter values, respectively, the relative risk indicating the risk for lack of glycemic control being higher for one of the first patient and the second patient

the data processing device further being configured to at least one of the following:

a) trigger an electronic message being sent to the first patient comprising a prompt to increase the measurement frequency if a ratio of the first total risk score and a predefined first maximum achievable total risk score is above a first predefined threshold, wherein the first predefined threshold is above 0.5, preferably above 0.7, more preferably above 0.8 or 0.9; and

b) trigger an electronic message being sent to the second patient comprising a prompt to increase the measurement frequency if a ratio of the second total risk score and a predefined second maximum achievable total risk score is above a second predefined threshold, wherein the second predefined threshold is above 0.5, preferably above 0.7, more preferably above 0.8 or 0.9.

9. A computer program product, comprising program code configured to, when loaded into a computer having one or more processors, perform the implemented method for determining a relative risk for lack of glycemic control for at least two patients according to at least one of the claims 1 to 7.

**Patentansprüche**

1. Computerimplementiertes Verfahren zum Bestimmen eines relativen Risikos für eine mangelnde glykämische Kontrolle bei mindestens zwei Patienten, wobei das Verfahren in einer Datenverarbeitungsvorrichtung (2) mit einem oder mehreren Datenprozessoren (3a) und einer Datenspeichervorrichtung (3b), die mit dem einen oder den mehreren Datenprozessoren (3a) verbunden ist, Folgendes umfasst:

- Bereitstellen eines ersten Satzes von Glukosemessdaten, die einem ersten Patienten zugeordnet sind, wobei der erste Satz von Glukosemessdaten durch Durchführen einer ersten Häufigkeit von Messereignissen gesammelt wird;

- Bereitstellen eines zweiten Satzes von Glukosemessdaten, die einem zweiten Patienten zugeordnet sind, wobei der zweite Satz von Glukosemessdaten durch Durchführen einer zweiten Häufigkeit von Messereignissen gesammelt wird, die sich von der ersten Häufigkeit von Messereignissen unterscheidet;

- Bereitstellen einer Vielzahl von Bewertungsparametern in der Datenspeichervorrichtung (3b), wobei jeder der Bewertungsparameter mindestens einer der ersten Häufigkeit von Messereignissen und der zweiten Häufigkeit von Messereignissen zugeordnet ist;

- Bestimmen, in Abhängigkeit von der ersten Häufigkeit von Messereignissen, einer Vielzahl von ersten Bewertungsparametern aus der Vielzahl von Bewertungsparametern in der Datenspeichervorrichtung (3b), wobei die Vielzahl von ersten Bewertungsparametern aus der folgenden Gruppe von Bewertungsparametern ausgewählt ist: niedriger Blutglukoseindex; hoher Blutglukoseindex, mittlerer Blutglukosewert und medianer Blutglukosewert;

- Bestimmen, in Abhängigkeit von der zweiten Häufigkeit von Messereignissen, einer Vielzahl von zweiten Bewertungsparametern aus der Vielzahl von Bewertungsparametern in der Datenspeichervorrichtung (3b), wobei mindestens einer der bestimmten Vielzahl von zweiten Bewertungsparametern sich von der Vielzahl von ersten Bewertungsparametern unterscheidet und wobei die Vielzahl von zweiten Bewertungsparametern aus der folgenden Gruppe von Bewertungsparametern ausgewählt ist: niedriger Blutglukoseindex; hoher Blutglukoseindex, Stabilitätsindex, der als Mittelwert des zweiten Satzes von Glukosemessdaten geteilt durch die Standardabweichung definiert ist, und mittlerer täglicher Risikobereich (ADRR);

- Bestimmen einer Vielzahl von ersten Bewertungsparameterwerten, die dem ersten Patienten für die Vielzahl von ersten Bewertungsparametern zugeordnet sind, aus dem ersten Satz von Glukosemessdaten;

- Bestimmen einer Vielzahl von zweiten Bewertungsparameterwerten, die dem zweiten Patienten für die Vielzahl von zweiten Bewertungsparametern zugeordnet sind, aus dem zweiten Satz von Glukosemessdaten;

- Bestimmen einer Vielzahl von ersten Risikowerten, die dem ersten Patienten zugeordnet sind, und einer Vielzahl von zweiten Risikowerten, die dem zweiten Patienten zugeordnet sind, aus der Vielzahl von ersten Bewertungsparameterwerten bzw. der Vielzahl von zweiten Bewertungsparameterwerten; und

- Bestimmen eines relativen Risikos für mangelnde glykämische Kontrolle durch Vergleichen eines ersten Gesamtrisikowerts und eines zweiten Gesamtrisikowerts, wobei der erste Gesamtrisikowert und der zweite Gesamtrisikowert durch Aufsummieren des ersten und zweiten Risikowerts für die Vielzahl von ersten Bewertungsparameterwerten bzw. die Vielzahl von zweiten Bewertungsparameterwerten bestimmt werden, wobei das relative Risiko anzeigt, dass das Risiko für mangelnde glykämische Kontrolle für einen des ersten Patienten und des zweiten Patienten höher ist,

- wobei das Verfahren ferner mindestens eines der Folgenden umfasst:

a) Vordefinieren eines ersten maximal erreichbaren Gesamtrisikowerts und eines ersten Schwellenwerts, wobei ein Verhältnis des ersten Gesamtrisikowerts und des ersten maximal erreichbaren Gesamtrisikowerts, das über dem ersten vordefinierten Schwellenwert liegt, veranlasst, dass eine elektronische Nachricht an den ersten Patienten gesendet wird, die eine Aufforderung zum Erhöhen der Messhäufigkeit umfasst, wobei der erste vordefinierte Schwellenwert über 0,5, vorzugsweise über 0,7, bevorzugter über 0,8 oder 0,9 liegt; und

b) Vordefinieren eines zweiten maximal erreichbaren Gesamtrisikowerts und eines zweiten Schwellenwerts, wobei ein Verhältnis des zweiten Gesamtrisikowerts und des zweiten maximal erreichbaren Gesamtrisikowerts, das über dem zweiten vordefinierten Schwellenwert liegt, veranlasst, dass eine elektronische Nachricht an den zweiten Patienten gesendet wird, die eine Aufforderung zum Erhöhen der Messhäufigkeit umfasst, wobei der zweite vordefinierte Schwellenwert über 0,5, vorzugsweise über 0,7, bevorzugter über 0,8 oder 0,9 liegt.

2. Verfahren nach Anspruch 1, umfassend mindestens eines der Folgenden:

- Bereitstellen eines ersten Satzes von Glukosemessdaten, die in einer ersten Vielzahl von Punktüberwachungs-Glukosemessereignissen unter Anwendung der ersten Häufigkeit von Messereignissen gesammelt werden; und
- Bereitstellen eines zweiten Satzes von Glukosemessdaten, die in einer zweiten Vielzahl von Punktüberwachungs-Glukosemessereignissen unter Anwendung der zweiten Häufigkeit von Messereignissen gesammelt werden.

3. Verfahren nach Anspruch 1 oder 2, umfassend für mindestens eines der ersten und zweiten Glukosemessdaten:

- Bestimmen eines oder mehrerer aufeinanderfolgender Glukosemesswerte, die nach dem Sammeln eines vorhergehenden Glukosemesswerts in einem vorhergehenden Messereignis gesammelt werden, wobei der eine oder die mehreren Messwerte in einem oder mehreren aufeinanderfolgenden Messereignissen innerhalb eines vorbestimmten Zeitfensters nach dem vorhergehenden Messereignis gesammelt werden; und
- Ausschließen des einen oder der mehreren aufeinanderfolgenden Glukosemesswerte aus dem Bestimmen des ersten/zweiten Bewertungsparameterwerts.

4. Verfahren nach Anspruch 1, wobei mindestens ein gemeinsamer Bewertungsparameter sowohl in der Vielzahl von ersten als auch der Vielzahl von zweiten Bewertungsparametern bereitgestellt wird.

5. Verfahren nach mindestens einem der vorhergehenden Ansprüche, wobei

- der erste Satz von Glukosemessdaten einem ersten Patienten mit einem ersten Typ von Diabetes zugeordnet ist; und
- der zweite Satz von Glukosemessdaten einem zweiten Patienten mit einem zweiten Typ von Diabetes zugeordnet ist;

ferner umfassend das Auswählen mindestens eines des mindestens einen ersten Bewertungsparameters und des mindestens einen zweiten Bewertungsparameters in Abhängigkeit von dem ersten Typ von Diabetes bzw. dem zweiten Typ von Diabetes.

6. Verfahren nach mindestens einem der vorhergehenden Ansprüche, wobei sich der zweite Patient von dem ersten Patienten unterscheidet.

7. Verfahren nach mindestens einem der vorhergehenden Ansprüche, wobei mindestens einer des ersten Gesamtrisikowerts und des zweiten Gesamtrisikowerts, die über einem vordefinierten Schwellenwert liegen, mindestens eines von einem Bedarf an einer Therapieüberprüfung und einer Therapieanpassung für den ersten Patienten bzw.

den zweiten Patienten anzeigt.

8. System zum Bestimmen eines relativen Risikos für eine mangelnde glykämische Kontrolle bei mindestens zwei Patienten, wobei das System eine Datenverarbeitungsvorrichtung (2) mit einem oder mehreren Datenprozessoren (3a) und einer Datenspeichervorrichtung (3b), die mit dem einen oder den mehreren Datenprozessoren (3a) verbunden ist, umfasst, wobei die Datenverarbeitungsvorrichtung (2) konfiguriert ist zum:

- Bereitstellen eines ersten Satzes von Glukosemessdaten, die einem ersten Patienten zugeordnet sind, wobei der erste Satz von Glukosemessdaten durch Durchführen einer ersten Häufigkeit von Messereignissen gesammelt wird;
- Bereitstellen eines zweiten Satzes von Glukosemessdaten, die einem zweiten Patienten zugeordnet sind, wobei der zweite Satz von Glukosemessdaten durch Durchführen einer zweiten Häufigkeit von Messereignissen gesammelt wird, die sich von der ersten Häufigkeit von Messereignissen unterscheidet;
- Bereitstellen einer Vielzahl von Bewertungsparametern in der Datenspeichervorrichtung (3b), wobei jeder der Bewertungsparameter mindestens einer der ersten Häufigkeit von Messereignissen und der zweiten Häufigkeit von Messereignissen zugeordnet ist;
- Bestimmen, in Abhängigkeit von der ersten Häufigkeit von Messereignissen, einer Vielzahl von ersten Bewertungsparametern aus der Vielzahl von Bewertungsparametern in der Datenspeichervorrichtung (3b), wobei die Vielzahl von ersten Bewertungsparametern aus der folgenden Gruppe von Bewertungsparametern ausgewählt ist: niedriger Blutglukoseindex; hoher Blutglukoseindex, mittlerer Blutglukosewert und medianer Blutglukosewert;
- Bestimmen, in Abhängigkeit von der zweiten Häufigkeit von Messereignissen, einer Vielzahl von zweiten Bewertungsparametern aus der Vielzahl von Bewertungsparametern in der Datenspeichervorrichtung (3b), wobei mindestens einer der bestimmten Vielzahl von zweiten Bewertungsparametern sich von der Vielzahl von ersten Bewertungsparametern unterscheidet und wobei die Vielzahl von zweiten Bewertungsparametern aus der folgenden Gruppe von Bewertungsparametern ausgewählt ist: niedriger Blutglukoseindex; hoher Blutglukoseindex, Stabilitätsindex, der als Mittelwert des zweiten Satzes von Glukosemessdaten geteilt durch die Standardabweichung definiert ist, und mittlerer täglicher Risikobereich (ADRR);
- Bestimmen einer Vielzahl von ersten Bewertungsparameterwerten, die dem ersten Patienten für die Vielzahl von ersten Bewertungsparametern zugeordnet sind, aus dem ersten Satz von Glukosemessdaten;
- Bestimmen einer Vielzahl von zweiten Bewertungsparameterwerten, die dem zweiten Patienten für die Vielzahl von zweiten Bewertungsparametern zugeordnet sind, aus dem zweiten Satz von Glukosemessdaten;
- Bestimmen einer Vielzahl von ersten Risikowerten, die dem ersten Patienten zugeordnet sind, und einer Vielzahl von zweiten Risikowerten, die dem zweiten Patienten zugeordnet sind, aus der Vielzahl von ersten Bewertungsparameterwerten bzw. der Vielzahl von zweiten Bewertungsparameterwerten; und
- Bestimmen eines relativen Risikos für mangelnde glykämische Kontrolle durch Vergleichen eines ersten Gesamtrisikowerts und eines zweiten Gesamtrisikowerts, wobei der erste Gesamtrisikowert und der zweite Gesamtrisikowert durch Aufsummieren des ersten und zweiten Risikowerts für die Vielzahl von ersten Bewertungsparameterwerten bzw. die Vielzahl von zweiten Bewertungsparameterwerten bestimmt werden, wobei das relative Risiko anzeigt, dass das Risiko für mangelnde glykämische Kontrolle für einen des ersten Patienten und des zweiten Patienten höher ist,

wobei die Datenverarbeitungsvorrichtung ferner für mindestens eines von Folgendem konfiguriert ist:

a) Veranlassen, dass eine elektronische Nachricht an den ersten Patienten gesendet wird, die eine Aufforderung zum Erhöhen der Messhäufigkeit umfasst, wenn ein Verhältnis des ersten Gesamtrisikowerts und eines vordefinierten ersten maximal erreichbaren Gesamtrisikowerts über einem ersten vordefinierten Schwellenwert liegt, wobei der erste vordefinierte Schwellenwert über 0,5, vorzugsweise über 0,7, bevorzugter über 0,8 oder 0,9 liegt; und

b) Veranlassen, dass eine elektronische Nachricht an den zweiten Patienten gesendet wird, die eine Aufforderung zum Erhöhen der Messhäufigkeit umfasst, wenn ein Verhältnis des zweiten Gesamtrisikowerts und eines vordefinierten zweiten maximal erreichbaren Gesamtrisikowerts über einem zweiten vordefinierten Schwellenwert liegt, wobei der zweite vordefinierte Schwellenwert über 0,5, vorzugsweise über 0,7, bevorzugter über 0,8 oder 0,9 liegt.

9. Computerprogrammprodukt, das einen Programmcode umfasst, der konfiguriert ist, wenn er in einen Computer mit einem oder mehreren Prozessoren geladen wird, das implementierte Verfahren zum Bestimmen eines relativen Risikos für mangelnde glykämische Kontrolle bei mindestens zwei Patienten nach mindestens einem der Ansprüche 1 bis 7 durchzuführen.

**Revendications**

1. Procédé mis en œuvre par ordinateur permettant de déterminer un risque relatif résultant d'une absence de contrôle glycémique pour au moins deux patients, le procédé, dans un dispositif de traitement de données (2) ayant un ou plusieurs processeurs de données (3a) et un dispositif de stockage de données (3b) connecté au ou aux processeurs de données (3a), comprenant

- la fourniture d'un premier ensemble de données de mesure de glucose attribuées à un premier patient, le premier ensemble de données de mesure de glucose étant collecté en effectuant une première fréquence d'événements de mesure ;
- la fourniture d'un deuxième ensemble de données de mesure de glucose attribuées à un deuxième patient, le deuxième ensemble de données de mesure de glucose étant collecté en effectuant une deuxième fréquence d'événements de mesure qui est différente de la première fréquence d'événements de mesure ;
- la fourniture d'une pluralité de paramètres d'évaluation dans le dispositif de stockage de données (3b), chacun des paramètres d'évaluation étant attribué à au moins une parmi la première fréquence d'événements de mesure et la deuxième fréquence d'événements de mesure ;
- la détermination, en fonction de la première fréquence d'événements de mesure, d'une pluralité de premiers paramètres d'évaluation provenant de la pluralité de paramètres d'évaluation dans le dispositif de stockage de données (3b), dans lequel la pluralité de premiers paramètres d'évaluation est choisie dans le groupe suivant de paramètres d'évaluation : indice d'hypoglycémie ; indice d'hyperglycémie, glycémie moyenne et glycémie médiane ;
- la détermination, en fonction de la deuxième fréquence d'événements de mesure, d'une pluralité de deuxièmes paramètres d'évaluation provenant de la pluralité de paramètres d'évaluation dans le dispositif de stockage de données (3b), dans lequel au moins un parmi la pluralité déterminée de deuxièmes paramètres d'évaluation est différent de la pluralité de premiers paramètres d'évaluation, et dans lequel la pluralité de deuxièmes paramètres d'évaluation est choisie dans le groupe suivant de paramètres d'évaluation : indice d'hypoglycémie ; indice d'hyperglycémie, indice de stabilité défini en tant que valeur moyenne du deuxième ensemble de données de mesure de glucose divisée par l'écart-type et plage de risque quotidienne moyenne (ADRR) ;
- la détermination, à partir du premier ensemble de données de mesure de glucose, d'une pluralité de premières valeurs de paramètres d'évaluation attribuées au premier patient pour la pluralité de premiers paramètres d'évaluation ;
- la détermination, à partir du deuxième ensemble de données de mesure de glucose, d'une pluralité de deuxièmes valeurs de paramètres d'évaluation attribuées au deuxième patient pour la pluralité de deuxièmes paramètres d'évaluation ;
- la détermination d'une pluralité de premiers scores de risque attribués au premier patient et d'une pluralité de deuxièmes scores de risque attribués au deuxième patient à partir de la pluralité de premières valeurs de paramètres d'évaluation et de la pluralité de deuxièmes valeurs de paramètres d'évaluation, respectivement ; et
- la détermination d'un risque relatif résultant d'une absence de contrôle glycémique en comparant un premier score de risque total et un deuxième score de risque total, dans lequel le premier score de risque total et le deuxième score de risque total sont déterminés en additionnant les premier et deuxième scores de risque pour la pluralité de premières valeurs de paramètres d'évaluation et la pluralité de deuxièmes valeurs de paramètres d'évaluation, respectivement, le risque relatif indiquant le risque résultant d'une absence de contrôle glycémique qui est supérieur pour un parmi le premier patient et le deuxième patient
- le procédé comprenant en outre au moins l'une des suivantes :

a) la prédéfinition d'un premier score de risque total maximal pouvant être atteint et d'une première valeur seuil, et un rapport du premier score de risque total et du premier score de risque total maximal pouvant être atteint étant au-dessus du premier seuil prédéfini déclenchant l'envoi d'un message électronique au premier patient comprenant une invite pour augmenter la fréquence de mesure, dans lequel le premier seuil prédéfini est au-dessus de 0,5, de préférence au-dessus de 0,7, plus préférablement au-dessus de 0,8 ou de 0,9 ; et
b) la prédéfinition d'un deuxième score de risque total maximal pouvant être atteint et d'une deuxième valeur seuil, et un rapport du deuxième score de risque total et du deuxième score de risque total maximal pouvant être atteint étant au-dessus du deuxième seuil prédéfini, déclenchant l'envoi d'un message électronique au deuxième patient comprenant une invite pour augmenter la fréquence de mesure, dans lequel le deuxième seuil prédéfini est au-dessus de 0,5, de préférence au-dessus de 0,7, plus préférablement au-dessus de 0,8 ou de 0,9.

2. Procédé selon la revendication 1, comprenant au moins l'une des suivantes :

- la fourniture d'un premier ensemble de données de mesure de glucose collectées dans une première pluralité d'événements de mesure de glucose de surveillance ponctuelle en appliquant la première fréquence d'événements de mesure ; et
- la fourniture d'un deuxième ensemble de données de mesure de glucose collectées dans une deuxième pluralité d'événements de mesure de glucose de surveillance ponctuelle en appliquant la deuxième fréquence d'événements de mesure.

3. Procédé selon la revendication 1 ou 2, comprenant, pour au moins une parmi les premières et deuxièmes données de mesure de glucose :

- la détermination d'une ou de plusieurs valeurs de mesure de glucose consécutives collectées consécutivement à la collecte d'une valeur de mesure de glucose précédente dans un événement de mesure précédent, la ou les valeurs de mesure étant collectées dans un ou plusieurs événements de mesure consécutifs au sein d'une fenêtre de temps prédéterminée consécutivement à l'événement de mesure précédent ; et
- l'exclusion de la ou des valeurs de mesure de glucose consécutives de la détermination de la première/deuxième valeur de paramètre d'évaluation.

4. Procédé selon la revendication 1, dans lequel au moins un paramètre d'évaluation courant est fourni à la fois dans la pluralité de premiers et la pluralité de deuxièmes paramètres d'évaluation.

5. Procédé selon au moins l'une des revendications précédentes, dans lequel

- le premier ensemble de données de mesure de glucose est attribué à un premier patient ayant un premier type de diabète ; et
- le deuxième ensemble de données de mesure de glucose est attribué à un deuxième patient ayant un deuxième type de diabète ;

comprenant en outre la sélection d'au moins un parmi l'au moins un premier paramètre d'évaluation et l'au moins un deuxième paramètre d'évaluation en fonction du premier type de diabète et du deuxième type de diabète, respectivement.

6. Procédé selon au moins l'une des revendications précédentes, dans lequel le deuxième patient est différent du premier patient.

7. Procédé selon au moins l'une des revendications précédentes, dans lequel au moins un parmi le premier score de risque total et le deuxième score de risque total, étant au-dessus d'une valeur seuil prédéfinie, est indicatif d'au moins un parmi un besoin de réexamen de la thérapie, et d'ajustement de la thérapie pour le premier patient et le deuxième patient, respectivement.

8. Système permettant de déterminer un risque relatif résultant d'une absence de contrôle glycémique pour au moins deux patients, le système comprenant un dispositif de traitement de données (2) ayant un ou plusieurs processeurs de données (3a) et un dispositif de stockage de données (3b) connecté au ou aux processeurs de données (3a), dans lequel le dispositif de traitement de données (2) est configuré pour

- fournir un premier ensemble de données de mesure de glucose attribuées à un premier patient, le premier ensemble de données de mesure de glucose étant collecté en effectuant une première fréquence d'événements de mesure ;
- fournir un deuxième ensemble de données de mesure de glucose attribuées à un deuxième patient, le deuxième ensemble de données de mesure de glucose étant collecté en effectuant une deuxième fréquence d'événements de mesure qui est différente de la première fréquence d'événements de mesure ;
- fournir une pluralité de paramètres d'évaluation dans le dispositif de stockage de données (3b), chacun des paramètres d'évaluation étant attribué à au moins une parmi la première fréquence d'événements de mesure et la deuxième fréquence d'événements de mesure ;
- déterminer, en fonction de la première fréquence d'événements de mesure, une pluralité de premiers paramètres d'évaluation provenant de la pluralité de paramètres d'évaluation dans le dispositif de stockage de données (3b), dans lequel la pluralité de premiers paramètres d'évaluation est choisie dans le groupe suivant de paramètres d'évaluation : indice d'hypoglycémie ; indice d'hyperglycémie, glycémie moyenne et glycémie médiane ;

- déterminer, en fonction de la deuxième fréquence d'événements de mesure, une pluralité de deuxièmes paramètres d'évaluation provenant de la pluralité de paramètres d'évaluation dans le dispositif de stockage de données (3b), dans lequel au moins un paramètre parmi la pluralité déterminée de deuxièmes paramètres d'évaluation est différent de la pluralité de premiers paramètres d'évaluation, et dans lequel la pluralité de deuxièmes paramètres d'évaluation est choisie dans le groupe suivant de paramètres d'évaluation : indice d'hypoglycémie ; indice d'hyperglycémie, indice de stabilité défini en tant que valeur moyenne du deuxième ensemble de données de mesure de glucose divisée par l'écart-type et plage de risque quotidienne moyenne (ADRR) ;

- déterminer, à partir du premier ensemble de données de mesure de glucose, une pluralité de premières valeurs de paramètres d'évaluation attribuées au premier patient pour la pluralité de premiers paramètres d'évaluation ;

- déterminer, à partir du deuxième ensemble de données de mesure de glucose, une pluralité de deuxièmes valeurs de paramètres d'évaluation attribuées au deuxième patient pour la pluralité de deuxièmes paramètres d'évaluation ;

- déterminer une pluralité de premiers scores de risque attribués au premier patient et d'une pluralité de deuxièmes scores de risque attribués au deuxième patient à partir de la pluralité de premières valeurs de paramètres d'évaluation et de la pluralité de deuxièmes valeurs de paramètres d'évaluation, respectivement ; et

- déterminer un risque relatif résultant d'une absence de contrôle glycémique en comparant un premier score de risque total et un deuxième score de risque total, dans lequel le premier score de risque total et le deuxième score de risque total sont déterminés en additionnant les premier et deuxième scores de risque pour la pluralité de premières valeurs de paramètres d'évaluation et la pluralité de deuxièmes valeurs de paramètres d'évaluation, respectivement, le risque relatif indiquant le risque résultant d'une absence de contrôle glycémique qui est supérieur pour un parmi le premier patient et le deuxième patient

le dispositif de traitement de données étant en outre configuré pour au moins l'un des suivants :

a) déclencher l'envoi d'un message électronique au premier patient comprenant une invite pour augmenter la fréquence de mesure si un rapport du premier score de risque total et d'un premier score de risque total maximal pouvant être atteint prédéfini est au-dessus d'un premier seuil prédéfini, dans lequel le premier seuil prédéfini est au-dessus de 0,5, de préférence au-dessus de 0,7, plus préférablement au-dessus de 0,8 ou de 0,9 ; et

b) déclencher l'envoi d'un message électronique au deuxième patient comprenant une invite pour augmenter la fréquence de mesure si un rapport du deuxième score de risque total et d'un deuxième score de risque total maximal pouvant être atteint prédéfini est au-dessus d'un deuxième seuil prédéfini, dans lequel le deuxième seuil prédéfini est au-dessus de 0,5, de préférence au-dessus de 0,7, plus préférablement au-dessus de 0,8 ou de 0,9.

9. Produit-programme d'ordinateur, comprenant un code de programme configuré pour, lorsqu'il est chargé dans un ordinateur ayant un ou plusieurs processeurs, effectuer le procédé mis en œuvre pour déterminer un risque relatif résultant d'une absence de contrôle glycémique pour au moins deux patients selon au moins l'une des revendications 1 à 7.

Fig. 1

5a　　　　　　　5b

10

20

30

40

Fig. 2

| Scores (Risk) | 0 (NO RISK) | 1 (LOW RISK) | 2 (MEDIUM RISK) | 3 (HIGH RISK) |
|---|---|---|---|---|
| LBGI | <1 | <2.5 | <5 | >5 |
| HBGI | <1 | <4.5 | <9 | >9 |
| SI | >3 | >2 & <3 | <2 & >1 | <1 |
| ADRR | <19 | >20 & <30 | >30 & <40 | >40 |
| median** (A1C) | <154 (<7.0 HbA1c) | >154 & <183 (>7.0 HbA1c) | >183 & <212 (>8.0 HbA1c) | >212 (>9.0 HbA1c) |

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090171589 A1
- US 20150095042 A1
- EP 5062615 B1
- WO 2016182972 A1
- US 20060253303 A1
- WO 2017030961 A1
- WO 2007081853 A2
- US 20130102867 A1
- US 20140188400 A1
- EP 2339953 A1
- WO 2007081853 A

### Non-patent literature cited in the description

- **KOVATCHEV et al.** Risk analysis of blood glucose data: a quantitative approach to optimizing the control of insulin dependent diabetes. *J. Theor. Med*, 2000, vol. 3, 1-10
- **KOVATCHEV**. Metrics for glycemic control from HbA1c to continuous glucose monitoring. *Nature Rev. Endocrinology*, 2017, vol. 13, 425-436
- **KOVATCHEV et al.** *Diabetes Care*, 2006, vol. 29 (11), 2433-2438
- **BUIJSSE et al.** Risk Assessment Tools for Identifying Individuals at Risk of Developing Type 2 Diabetes. *Epidemiologic Review*, vol. 33 (1), 46-62